# EUROPÄISCHE PATENTSCHRIFT

(11) **EP 2 268 294 B1**
(45) Veröffentlichungstag und Bekanntmachung des Hinweises auf die Patenterteilung: **26.12.2012**
(21) Anmeldenummer: 09738117.2
(22) Anmeldetag: 27.04.2009
(51) Int. Cl.: A61K 35/64, A61P 31/12

(54) **PROPOLIS-EXTRAKT**
PROPOLIS EXTRACT
EXTRAIT DE PROPOLIS

(30) Priorität: 30.04.2008 DE 102008021756
(43) Veröffentlichungstag der Anmeldung: 05.01.2011
(73) Patentinhaber: Gehrlicher Pharmazeutische Extrakte GmbH, 82547 Eurasburg (DE)
(72) Erfinder: SENSCH, Karl-Heinz, 82547 Eurasburg (DE)
(74) Vertreter: von Kreisler Selting Werner
(86) Internationale Anmeldenummer: PCT/EP2009/055071
(87) Internationale Veröffentlichungsnummer: WO 2009/133073

(56) Entgegenhaltungen:
- WO-A-2005/094853
- DATABASE WPI Week 200404 Thomson Scientific, London, GB; AN 2004-039155 XP002544806 & JP 2003 335688 A (FANKERU KK) 25. November 2003 (2003-11-25)
- MIYATAKA HIDEKI ET AL: "Evaluation of propolis (II): Effects of Brazilian and Chinese propolis on histamine release from rat peritoneal mast cells induced by compound 48/80 and concanavalin A" BIOLOGICAL & PHARMACEUTICAL BULLETIN (OF JAPAN), PHARMACEUTICAL SOCIETY OF JAPAN, TOKYO, JP, Bd. 21, Nr. 7, 1. Juli 1998 (1998-07-01), Seiten 723-729, XP009122307 ISSN: 0918-6158
- NOLKEMPER S ET AL: "Mechanism of herpes simplex virus type 2 suppression by propolis extracts", PHYTOMEDICINE, GUSTAV FISCHER VERLAG, STUTTGART, DE, vol. 17, no. 2, 1 February 2010 (2010-02-01), pages 132-138, XP026832085, ISSN: 0944-7113 [retrieved on 2009-08-13]
- DATABASE MEDLINE [Online] US NATIONAL LIBRARY OF MEDICINE (NLM), BETHESDA, MD, US Januar 2010 SCHNITZLER PAUL ET AL: 'Antiviral activity and mode of action of propolis extracts and selected compounds.' Database accession no. NLM19472427 & SCHNITZLER PAUL ET AL: "Antiviral activity and mode of action of propolis extracts and selected compounds.", PHYTOTHERAPY RESEARCH : PTR JAN 2010 LNKD- PUBMED:19472427, vol. 24 Suppl 1, January 2010 (2010-01), pages S20-S28, ISSN: 1099-1573

## Beschreibung

Gegenstand der vorliegenden Erfindung ist ein Verfahren zur Herstellung eines hypoallergenen und darüber hinaus besonders wirksamen Propolis-Extraktes, die Verwendung dieses Extraktes sowie diesen Extrakt enthaltende pharmazeutische Zusammensetzungen.

Propolis (v. griech. προ "vor" und πολιζ "Stadt" - wegen des Vorkommens vornehmlich an den Fluglöchern von Bienenstöcken), auch Bienenharz, Bienenleim, Bienenkittharz, Kittharz oder Kittwachs genannt, ist ein starkes, natürlich vorkommendes Antibiotikum und auch Antimykotikum, ein Gemisch aus vielen unterschiedlichen Stoffen, deren Zusammensetzung stark variieren kann.

Da in einem Bienenstock die Insekten auf engstem Raum bei etwa 35 °C zusammenleben, herrschen dort im Grunde ideale Bedingungen für die Ausbreitung von Krankheiten. Deshalb dient das Material mit seiner Verwendung zum Abdichten von kleineren Öffnungen, Spalten und Ritzen in erster Linie dazu, Bakterien, Pilze und andere Mikroorganismen, die in den Stock eingeschleppt werden könnten oder vorhanden sind, in ihrer Entwicklung zu hemmen oder sogar abzutöten. Hierzu werden Oberflächen, beispielsweise auch das Innere der Wabenzellen für die Brut, mit einem hauchdünnen Propolisfilm überzogen.

Der Grundstoff für Propolis wird von Honigbienen als harzige Substanz an Knospen und teilweise auch an Wunden verschiedener Bäume (hauptsächlich Birken, Buchen, Erlen, Fichten, Pappeln, Rosskastanien und Ulmen) gesammelt (etwa 55 % Naturharz und Pollenbalsam). Weiterverarbeitet, mit etwa 30 % Wachs, etwa 5 % Pollenanteilen, etwa 10 % ätherischen Ölen aus den Blütenknospen und Speichelsekret (Fermenten) angereichert, handelt es sich um ein bei Stocktemperatur klebriges Baumaterial, das oft noch mit Bienenteilen und kleinsten Holzstücken verunreinigt ist.

Zur Gewinnung von Propolis kann der Imker an verschiedenen Stellen des Bienenkastens (Magazin-Beute), an den Bienen Ritzen oder ähnliches verkittet wurden, das Propolis abkratzen. Gezielter kann Propolis durch das Auflegen eines speziellen feinmaschigen Kunststoffgitters gewonnen werden. Die Bienen verkitten diese störenden Zwischenräume. Das Gitter wird danach entnommen und in den Gefrierschrank gelegt. Bei diesen tiefen Temperaturen ist Propolis dann sehr spröde und springt beim leichten Biegen des Kunststoffgitters von diesem ab.

Die Verwendung von Propolis beim Menschen reicht bis ins alte Ägypten zurück, wo es bei der Einbalsamierung der Mumien eingesetzt wurde. Im zweiten Weltkrieg wurde Propolis zur Wundbehandlung der Soldaten verwendet. Das Anwendungsspektrum von Propolis hat sich in der jüngeren Zeit jedoch erheblich erweitert, wie "Propolis - Wächter der Gesundheit" (erschienen in "Volksgesundheit" 4/84, Albert Aman-Verlag, Amorbach) entnommen werden kann. Dort heißt es:

"In zahlreichen Untersuchungen erwies sich Propolis als hochwirksames natürliches Antibiotikum mit breitem Wirkungsspektrum. Unter anderem bekämpft es zahlreiche Bakterien, Pilze und sogar verschiedene Viren, gegen die der Schulmedizin noch keine wirksamen Arzneimittel zur Verfügung stehen. Vor allem Eitererreger und Pilze, die Haut- und Schleimhautentzündungen hervorrufen, werden durch Propolis günstig beeinflusst. Ferner wurde nachgewiesen, dass Bienenkittharz Erkältung und Grippe vorbeugen kann und sogar gegen Infektionen mit Herpes-Viren, die neuerdings insbesondere im Bereich der Geschlechtsorgane erheblich zunehmen, aber auch den unangenehmen Bläschenausschlag im Mund-LippenBereich und auf der Mundschleimhaut sowie die Gürtelrose hervorrufen, gut wirkt.

Ein weiteres wichtiges Anwendungsgebiet sind schlecht heilende Verbrennungen, Wunden, Geschwüre und Hautentzündungen, insbesondere auch bei Akne, die nicht nur junge Menschen während der Pubertät stark belastet. In solchen Fällen wirkt Propolis oftmals erstaunlich schnell, selbst dann noch, wenn die schulmedizinische Therapie durch Antibiotika und Cortison nicht zufriedenstellend hilft. Und selbst die hartnäckige Schuppenflechte spricht auf Propolis gut an, sofern man sie durch eine Spezialdiät ergänzt.

Gute Erfahrungen konnten inzwischen auch bei Erkrankungen im Hals-Nasen-Ohren- und Augenbereich gesammelt werden. Mundgeruch, Zahnfleischentzündungen und -schwund, Zahnschmerzen durch Karies (aber nur zur Soforthilfe bis zur Konsultation des Zahnarztes), Rachen-, Mandel- und Kehlkopfentzündungen gehören ebenso wie Entzündungen und Abszesse des äußeren Gehörganges, Bindehaut-, Lidrandentzündungen und das Gerstenkorn zu den Anwendungsgebieten. Zur Vorbeugung von Karies gibt es neuerdings auch eine Zahnpasta mit Propoliszusatz. [...]

Schließlich kann Propolis auch noch bei zahlreichen inneren Erkrankungen allein oder ergänzend verwendet werden. Besonders gut wirkt das Kittharz auf den Darm. Viele Infektionen und Entzündungen lassen sich dank der antibiotischen und entzündungshemmenden Wirkung heilen, ohne dass die Darmkeime zerstört werden. Aber auch zur Nachbehandlung des Darms nach Antibiotika- und Strahlentherapie, bei Darmträgheit, Durchfall oder häufigem Wechsel von Durchfall und Verstopfung bewährten sich die "Gesundheitswächter". Ferner kommt Propolis bei Erkrankungen anderer Verdauungsorgane in Betracht, wie Entzündungen und Geschwüre des Magens, Leberentzündungen, Gelbsucht und andere Leberleiden.

Ebenso günstig werden Reizungen und Entzündungen der Nieren, Harnwege, Harnblase und Vorsteherdrüse beeinflusst. Für Frauen empfiehlt sich Propolis nach Erfahrungen, die bis in die Antike zurückreichen, bei Menstruationsstörungen, Entzündungen der Eierstöcke, Eileiter und Scheide (bakteriell oder durch Pilze) und gegen Ausfluss."

Wie einer Stellungnahme des Bundesinstituts für gesundheitlichen Verbraucherschutz und Veterinärmedizin, Deutschland vom September 2001 entnommen werden kann, sind Propolis und Propolis enthaltende Produkte jedoch nicht vollkommen unbedenklich.

Im Zusammenhang mit Propolis sind sowohl bei Anwendung als Arzneimittel als auch bei äußerlicher Anwendung in kosmetischen Mitteln (kontakt-) allergische Reaktionen beschrieben worden. Als allergen können die Inhaltsstoffe 1,1-Dimethylallyl- und Phenylethyl-3,3-dihydroxycinnamat und Benzylsalicylat eingestuft werden (Schumann und Grunow: Pflanzliche Allergene in kosmetischen Mitteln - das Beispiel Propolis. Bundesgesundheitsblatt 34 (1991) 11-12), welche sich vorwiegend in den Wachs- und Harzanteilen des Propolis befinden. In Propolis sind mehr als 150 verschiedene Substanzen identifiziert worden, darunter Terpene, Phenylpropanderivate und Flavonoide (Volpert- und Elstner: Biochemical activities of propolis extracts I. Standardization and antioxidative properties of ethanolic and aqueous derivatives. Z. Naturforsch. 48c: 851-857 (1993)).

Es verwundert daher nicht, dass schon erhebliche Anstrengungen unternommen worden sind, Propolis-Extrakte herzustellen, welche keine oder einen erheblich verminderten Anteil der allergenen Substanzen enthalten.

So beschreibt beispielsweise DE 692 28 966 T2 ein Verfahren zur Herstellung eines gereinigten Propolis-Extrakts und dessen Verwendungen. Das Verfahren umfasst dabei vorzugsweise die folgenden Schritte:

Bereitstellung einer Lösung eines rohen Propolis-Extrakts in einem Lösungsmittelgemisch von mindestens 70 V/V-% lösliches organisches Lösungsmittel in Wasser, Herabsetzen des Gehalts des wasserlöslichen organischen Lösungsmittels im Lösungsmittelgemisch auf eine Menge im Bereich von 30 bis 55 V/V-%, Stehenlassen der erhaltenen Lösung bei Umgebungstemperatur von 0,5 bis 20 Stunden zur Bildung einer oberen Schicht, die die gelösten Propolisbestandteile enthält und einer unteren Flüssigkeitsschicht, die ein Sediment enthält.

Die japanische Patentoffenlegung Nr. 197,523/86 schlägt ein Verfahren zum Entwachsen eines Propolis-Extrakts mit Ether als Versuch zur Überwindung der oben erwähnten Nachteile vor. Das Verfahren umfasst die Extraktion einer rohen Propolisprobe mit einem in Wasser leicht löslichen organischen Lösungsmittel, wie Ethanol, das Einengen des erhaltenen Extrakts und das Versetzen des erhaltenen Konzentrats mit Ether, um die Wachse abzutrennen und zu entfernen.

Dieses Verfahren weist allerdings die folgenden Nachteile auf: Es erfordert unbedingt eine Stufe, um das leicht wasserlösliche organische Lösungsmittel zu verdampfen, und dieses führt zu einem überaus starken Energieverbrauch und einem komplizierten Verfahren, wobei ebenso die Gefahr von Feuer einhergeht, weil Ether mit einem relativ niedrigen Siedepunkt verwendet wird.

Die japanische Patentoffenlegung Nr. 165,595/89 schlägt ein Verfahren zur Behandlung von Propolis mit Aktivkohle und/oder einem Kationenaustauscherharz zum Zwecke der Adsorption vor.

Das Verfahren umfasst das Extrahieren einer rohen Propolisprobe mit einem leicht wasserlöslichen organischen Lösungsmittel, wie Ethanol, das sofortige Versetzen mit Aktivkohle und/oder einem Kationenaustauscherharz zu dem erhaltenen Extrakt und die Abtrennung der wirksamen Bestandteile des Propolis durch Filtration.

Das Verfahren weist allerdings die folgenden Nachteile auf: Es erfordert eine relativ große Menge Aktivkohle und/oder Kationenaustauscherharz, um die Färbung, das Aroma und den Geschmack des Propolis zufriedenstellend zu verbessern.

RU 2139035 C1 beschreibt die Herstellung eines Mundwassers, welches einen wasserlöslichen Propolis-Extrakt als Ersatz für sonst übliche Extrakte aus Granatapfelfruchtschalen oder Süßholzwurzel zur Behandlung und Vorbeugung von Parodontose enthält. An Tierversuchen konnte eine entzündungshemmende Wirkung nachgewiesen werden. Um den Propolis-Extrakt zu erhalten werden aufwändige Reinigungsschritte beschrieben. Nach der Mischung des gemahlenen Propolis mit Alkohol sind mehrere Filtrationen, konstantes Rühren und Destillation nötig.

Auch JP-11-290005 A und JP 10-338641 A beschäftigen sich mit der Herstellung eines wasserlöslichen Propolis-Extraktes. In beiden Fällen findet eine erste Extraktion mit Hilfe einer ethanolischen Lösung statt, anschließend sind aufwändige Reinigungsschritte erforderlich. In JP-11-290005 A umfasst die Aufreinigung die Zugabe von Diatomenerde, Filtrationen, eine Gefriertrocknung und eine Aufreinigung des gefriergetrockneten Produktes über eine Gelfiltration. Auch in JP 10-338641 A fallen aufwändige Reinigungsschritte in Form von Fraktionierung, Separierung, Zentrifugierung und Filtration an.

Durch eine Hemmung der Aktivität von Hyaluronidase wird in JP-11-290005 A eine antibakterielle Wirkung erklärt. Hyaluronidase senkt die Viskosität von Hyaluronsäure wodurch die Durchlässigkeit von Gewebe erhöht wird. Bakterien, wie Staphylococcus aureus oder Staphylococcus pyogenes stellen Hyaluronidase her, wodurch sie eine erhöhte Mobilität im Gewebe erreichen. Propolis-Extrakt soll dem entgegenwirken können.

Neben der antibakteriellen Wirkung ist auch eine antivirale Wirkung von Propolis-Extrakten oder von Propolis-Material grundsätzlich bekannt. Harish et al. (Drugs Exptl. Clin. Res. XXIII (2) 89-96 (1997)) beschreibt einen unterdrückenden Einfluss auf die Replikation von HI-Viren vom Typ 1 durch Propolis und die Herstellung eines Propolis-Extraktes. Die Extraktion des Propolis-Rohmaterials erfolgt mit einer hochprozentige ethanolischen Lösung. Die unlöslichen Präzipitate werden durch Zentrifugation entfernt und anschließend durch exakt definierte Filtrationsschritte und Lyophilisierung gereinigt.

Es ist mit den im Stand der Technik beschriebenen Verfahren jedoch nicht möglich, auf einfache Art und Weise einen hypoallergenen Extrakt herzustellen, bei dem die für allergische Reaktionen und Hautirritationen verantwortlichen Wachs- und Harzanteile ohne Zugabe weiterer Zusatzstoffe nahezu vollständig entfernt werden.

Es ist somit eine Aufgabe der vorliegenden Erfindung ein Verfahren bereitzustellen, das die oben beschriebenen Nachteile des Standes der Technik vermeidet; insbesondere ist es eine Aufgabe der vorliegenden Erfindung, eine Aufreinigung eines Propolis-Rohstoffes zu entwickeln, so dass ohne Verwendung von Zusatzstoffen neben dem Extraktionsmittel selbst der Anteil der allergenen Substanzen deutlich vermindert wird und der erhaltene Extrakt als hypoallergen eingestuft werden kann.

Des Weiteren sollen die Gehalte an medizinisch wirksamen Substanzen im anfallenden Extrakt im Vergleich zu Extrakten des Standes der Technik erhöht sein.

Vor allem die im Propolis-Rohstoff enthaltenen Wachs- und Harzanteile sind für allergische Reaktionen und Hautirritationen verantwortlich. Diese treten in einem Masse- und Volumenmäßig hohen Anteil im Propolis-Rohstoff auf. Die Abtrennung dieser Bestandteile stellt einen bedeutenden Verfahrensschritt dar. Es sollen auf einfache Weise lediglich die Wachs- und Harzanteile entfernt werden, nicht jedoch die wirksamen Bestandteile, deren Konzentration angereichert werden soll.

Auch ist es eine weitere Aufgabe der vorliegenden Erfindung einen solchen Propolis-Extrakt bereitzustellen, der gegen virale Erkrankungen und/oder Entzündungen eine höhere Wirksamkeit zeigt als dies bei Extrakten des Standes der Technik der Fall ist.

Gelöst wird die der vorliegenden Erfindung zugrundeliegende Aufgabe in einer ersten Ausführungsform durch ein Verfahren zur Herstellung eines Extraktes aus einem Propolis-Rohstoff, umfassend die folgenden Schritte:
a) Extraktion des Propolis-Rohstoffes mit einem organischen Lösungsmittel enthaltenden Extraktionsmittel bei einer gegenüber der Raumtemperatur erhöhten Temperatur **oberhalb von 25 °C,**
b) Absenkung der Temperatur der in Schritt a) erhaltenen Lösung auf Raumtemperatur **in einem Bereich von 15 bis 25 °C,**
c) Abtrennung der in Schritt b) gebildeten unlöslichen Stoffe bei Raumtemperatur,
d) Absenkung der Temperatur der in Schritt c) erhaltenen Lösung auf eine Temperatur **unterhalb von 0 °C,**
e) Abtrennung der in Schritt d) gebildeten unlöslichen Stoffe, und
f) teilweise oder vollständige Entfernung des Extraktionsmittels.

Unter Propolis-Rohstoff ist dabei natürliches, das heißt jegliches aus der Natur gewinnbare Propolis zu verstehen.

Unter Raumtemperatur zu verstehen ist eine in mittleren Breitengraden auftretende Durchschnittstemperatur, das heißt eine Temperatur in einem Bereich von 15 bis 25 °C, insbesondere 20 °C.

Durch dieses erfindungsgemäße Verfahren wird erreicht, dass allergene Stoffe, insbesondere Harze und Wachse, in Schritt d) als unlösliche Stoffe anfallen und leicht vom Rest des die eigentlichen gewünschten Wirkstoffe enthaltenden Extraktes insbesondere im Tiefkühlverfahren abgetrennt werden können.

Im Gegensatz zu anderen Verfahren des Standes der Technik hat es sich im Rahmen des erfindungsgemäßen Verfahrens überraschend gezeigt, dass die Menge der wirksamen und daher erwünschten Substanzen durch die Abfolge der Schritte a) bis f) nicht signifikant vermindert wird bzw. bezogen auf den Propolis-Rohstoff erhöht wird. Die Gehalte an medizinisch wirksamen Verbindungen sind im resultierenden Extrakt dementsprechend höher als in herkömmlichen Propolis-Extrakten. Unter medizinisch wirksamen Verbindungen werden im Sinne der vorliegenden Erfindung vor allem die Flavonoide Quercetin, Chrysin, Pinocembrin und Galangin und Apigenin sowie die Phenolcarbonsäuren Kaffeesäure, p-Cumarsäure, Benzoesäure, Zimtsäure und Ferulasäure verstanden.

Das oben beschriebene Verfahren geht bevorzugt mit einer analytischen Standardisierung einher, die einen - in einer Vielzahl von analytisch detektierbaren Verbindungen - immer gleichen Propolis-Extrakt resultieren lässt.

Die in Schritt f) beschriebene Entfernung des Extraktionsmittels wird bevorzugt so weit vorgenommen, dass eine homogener Spissum- (also viskoser) Extrakt resultiert, welche sehr leicht galenisch in Öl-in-Wasser- und Wasser-in-Öl-Emulsionen einzuarbeiten ist. Solche homogenen Spissum-Extrakte haben gegenüber schwerlöslichem Roh-Propolis oder anderen, meist inhomogenen Propolis-Extrakten also klare Vorteile.

Alternativ lässt sich die Entfernung des Extraktionsmittels derart steuern, dass man einen Trocken- oder Flüssigextrakt erhält.

Bevorzugt setzt man als Extraktionsmittel ein organisches Lösungsmittel mit einem verzweigten oder unverzweigten Alkohol einer Kettenlänge von 1 bis 5 C-Atomen ein. Solche alkoholischen Lösungsmittel sind dabei insofern bevorzugt, als sie eine besonders hohe Löslichkeit für die eigentlich medizinisch wirksamen Substanzen aufweisen, weshalb zur eigentlichen Extraktion verhältnismäßig wenig Lösungsmittel verwendet werden muss. Ferner ist zwar die Löslichkeit auch der allergenen Substanzen in solchen Lösungsmitteln ausreichend, doch lassen sich diese bei Verwendung eben dieser Lösungsmittel sehr leicht durch eine Absenkung der Temperatur in Schritt d) abtrennen.

Ganz besonders bevorzugt führt man die Extraktion mit einem Ethanol/Wasser-Gemisch durch, welches einen Ethanol-Gehalt in einem Bereich von 10 bis 90 Vol.-% aufweist. Die Verwendung von Ethanol als organisches Lösungsmittel stellt dabei insofern einen Vorteil dar, als dass dieser aus dem späteren Extrakt nicht notwendigerweise vollständig entfernt zu werden braucht (aufgrund seiner geringen Toxizität und aufgrund seiner zusätzlichen antibiotischen Wirkungsweise). Auch hat sich dieses Extraktionsgemisch als bevorzugt herausgestellt, da sich mit ihm ein sehr homogener Spissum-Extrakt erhalten lässt.

Die Temperatur der in Schritt c) erhaltenen Lösung wird in Schritt d) unterhalb von 0 °C, insbesondere in einem Bereich von -5 bis -30 °C abgesenkt. Diese Temperaturbereiche gelten als besonders bevorzugt, da sie eine sehr gute Abtrennung der allergenen Stoffe bei einer Beibehaltung der medizinisch erwünschten Stoffe in Lösung gestatten. Die Einstellung einer definierten Temperatur zur Ausfällung der unlöslichen Stoffe richtet sich dabei im Wesentlichen nach der Konzentration der in den Schritten a) bis c) erhaltenen Lösung.

Daneben führt man die Ausfällung in Schritt d) bevorzugt in einem Zeitfenster von 1 bis 48 h durch. Geringere Zeitdauern haben sich insofern nicht bewährt, als eine nur unvollständige Ausfällung der abzutrennenden Stoffe erzielt wird. Längere Zeitdauern führen nicht nur zu keiner signifikanten Verbesserung der Quantität der ausgefallenen Stoffe sondern bedingt auch höhere Kosten durch das längere Aufrechterhalten von Temperaturen unterhalb Raumtemperatur.

Die so gebildeten unlöslichen Stoffe werden bevorzugt abfiltriert, da dadurch einfach und effektiv eine gute Abtrennung der unlöslichen Stoffe gewährleistet wird. Zum Einsatz kommen können hier verschiedenste Papierfilter und/oder Keramikfilter oder weitere im Stand der Technik hinlänglich bekannte Filtermaterialien.

Das erfindungsgemäße Verfahren weist einen Abtrennungsschritt auf, der im Anschluss an Schritt b), also noch vor der Tieftemperaturbehandlung durchgeführt wird. Nach der Extraktion wird der erhaltene Extrakt zunächst auf Raumtemperatur abgekühlt. An dieser Stelle zeigt sich in der Regel schon eine erste ausgefällte 'Fraktion' unlöslicher Stoffe, welche zunächst abgetrennt, insbesondere abfiltriert wird.

Die Entfernung des Extraktionsmittels wird bevorzugt bei einem Druck unterhalb des 'normalen' Luftdrucks, das heißt unterhalb von 500 mbar durchgeführt. Als besonders bevorzugt haben sich hier Drücke von unterhalb von 150 mbar herausgestellt. Dies gestattet eine schonendere Abtrennung des Extraktionsmittels bei einer geringeren Temperatur.

Bei dem organische Lösungsmittel enthaltenden Extraktionsmittel kann es sich ebenfalls um Kohlendioxid handeln. Es kommt also hier eine Extraktion unter Verwendung überkritischen Kohlendioxids zur Anwendung, bei dem lediglich die Druckbereiche so gewählt werden müssen, dass unter den gegebenen (Temperatur-) Bedingungen überkritisches Kohlendioxid vorliegt. Ansonsten braucht an den erfindungsgemäßen Verfahrensschritten jedoch keine Änderung vorgenommen werden.

Überraschenderweise hat sich gezeigt, dass die Aufreinigung ohne weitere Hilfsmittel entgegen der Auffassung des Standes der Technik möglich ist. Entscheidend ist die Einstellung der Temperatur im Anschluss an die Extraktion. Bei der Abkühlung zunächst auf Raumtemperatur (25 °C) und anschließend auf unter Raumtemperatur, bevorzugt auf -5 °C bis -30 °C, werden die allergenen Harz- und Wachsanteile entfernt, wie in Schritten d) und e) beschrieben ; die antiviralen Bestandteile bleiben jedoch im Extrakt erhalten.

In einer weiteren Ausführungsform wird die der Erfindung zugrundeliegende Aufgabe gelöst durch einen nach dem erfindungsgemäßen Verfahren erhältlichen Propolis-Extrakt zur Behandlung viraler Erkrankungen und/oder Entzündungen, insbesondere Herpes.

So wurde ein nach dem oben beschriebenen Verfahren hergestellter Propolis-Extrakt nach phytochemischer Standardisierung auf seine viruziden Eigenschaften getestet. Im *in vitro* Vergleich mit bekannten Mitteln wie Heparin-Natrium, Glycosaminglycan Polysulfat und Zinksulfat zeigten sich mit dem erfindungsgemäßen Propolis-Extrakt hochsignifikante erfolgreiche Ergebnisse - sowohl gegen den Herpes-Virus I, den Herpes-Virus II als auch den Herpes-Zoster-Virus.

Gerade bei der Behandlung von Herpes, welche in der Regel eine äußerlich Anwendung des Extraktes erforderlich macht, kommen die besonders vorteilhaften Eigenschaften des erfindungsgemäßen Extraktes besonders deutlich zum Vorschein: Aufgrund der Anreicherung der medizinisch wirksamen Substanzen, das heißt insbesondere der Flavonoide und der Phenolcarbonsäuren, besitzt ein erfindungsgemäßer Extrakt eine besonders hohe Wirksamkeit. Aufgrund des Fehlens der allergenen Substanzen kann der Extrakt als hypoallergen eingestuft werden, weist also nur ein sehr geringes allergenes Potential auf.

Zur Behandlung von Herpes-Erkrankungen kommt der Extrakt günstigerweise als Gel, Lotion, Emulsion, Lippenbalsam oder Lippencrème oder in Form einer sonstigen auf den Lippen und auf betroffene Hautpartien bei Herpes zoster und Herpes genitalis applizierbaren Form zur Anwendung.

Pharmazeutischen Zusammensetzungen enthalten den erfindungsgemäßen Extrakt bevorzugt in einer Menge von 0,1 bis 2,0 Gew.-%, insbesondere in einer Menge von 0,5 Gew.-%. Unterhalb dieser Untergrenze von 0,1 Gew.-% zeigt sich eine nur unzureichende antivirale Wirkung. Höhere Dosen als 2 Gew.-% sollten vermieden werden, da es sich in klinischen Studien gezeigt hat, dass die Wahrscheinlichkeit lokal reizender Reaktionen bei höheren Wirkstoffgehalten wieder signifikant zunimmt.

In einer dritten Ausführungsform wird die der Erfindung zugrundeliegende Aufgabe gelöst durch pharmazeutische Zusammensetzungen, die einen Propolis-Extrakt enthalten, wie er nach dem erfindungsgemäßen Verfahren zugänglich ist.

Die hypoallergene Wirksamkeit des Propolis-Extraktes konnte in einer Studie nachgewiesen werden. Bei einer dermatologischen Studie an 300 Patienten, welche mit Herpes labiles 1 infiziert waren, gab es keinerlei Hinweise auf allergische Reaktionen.

Um eine gleichbleibende Qualität und Standardisierung des Propolis-Extraktes zu gewährleisten, wird dieser vorzugsweise aus Propolis-Rohstoffen Mitteleuropas, insbesondere dem böhmisch-mährischen Gebiet, hergestellt.

### Ausführungsbeispiel:

Die Propolis Rohdroge wurde nach Eingangskontrolle und Freigabe durch das analytische Laboratorium min. 12 Stunden bei 30°C-50°C mazeriert und dann mit dem organischen Lösungsmittel extrahiert. Als Extraktionsmittel diente Ethanol 70% V/V, wobei ein Teil Propolis-Rohstoff mit fünf Teilen Extraktionsmittel angesetzt wurden.

Nach der Mazeration erfolgte eine Abkühlungsphase auf Raumtemperatur (vorzugsweise zwischen 15°C und 25°C). In dieser Abkühlungsphase über 12-24 Stunden erfolgte die Sedimentation und auch Präzipitation von hydrophilen Bestandteilen, die sich als Sediment ablagern.

Danach wurde die überstehende Flüssigkeit dekantiert und eine Filtration durchgeführt.

Das Filtrat wurde im Tiefkühlverfahren zwischen -5°C und -30°C (vorzugsweise zwischen -12°C und -25°C) über mind. 12 Stunden eingelagert.

In dieser Herstellungsphase erfolgte die Präzipitation und Sedimentation und damit die Abtrennung der Wachs- und Harzbestandteile.

Die überstehende Flüssigkeit wurde erneut einer Filtration unterworfen. Das Filtrat wurde im Vakuum zu einem homogenen Spissumextrakt eingeengt.

Die folgende Tabelle zeigt die allgemeine Spezifikation des erfindungsgemäßen Propolis-Extraktes sowie die Analyse eines nach dem erfindungsgemäßen Verfahren hergestellten Extraktes:

| Extraktionsmittel: Ethanol 70 V/V h. Eur. | | |
|---|---|---|
| Qualität | Spezifikation | Ergebnisse |
| Konsistenz | > 40°C geschmeidig, klebrig/< 15 °C fest, spröde | entspricht |
| Organoleptik | Farbe: braun | entspricht |
| | Geruch: aromatisch, charakteristisch, balsamisch | |
| | Geschmack: aromatisch, wachsig, bitter | |
| Identität: | Vergleichsspektrum | entspricht |
| Spektrum UV-VIS: | | |
| DC-Fingerprint: | Vergleichsspektrum | entspricht |
| HPLC-Fingerprint | Vergleichsspektrum | entspricht |
| Reinheit: | 2,5 bis 5,0 | 3,7 |
| pH-Wert Ph. Eur.: (10 % wässrig) | | |
| Asche Ph. Eur.: | < 0,5 % m/m | 0,22 % m/m |
| Trockenrückstand Ph. Eur.: | 80 bis 90 m/m | 81,8 % m/m |
| Wassergehalt nach | < 8 % m/m | 4,10 % m/m |
| Karl Fischer Ph. Eur.: | | |
| Brechunqsindex Ph. Eur.: | 1,500 bis 1,650 | 1,564 |
| Wachse | < 5 m/m | entspricht < 5 % m/m |
| Ethanolqehalt (GC): | < 5 % V/V | 1,3 % V/V |
| Gehalt: | > 5,0 % m/m | 11,7 % m/m |
| Polyphenole | | |
| Flavonoide | | |
| Quercetin : | ≥ 0,05 % m/m | 0,15 % m/m |
| Chrysin (HPLC) : | ≥ 2,0 % m/m | 3,07 % m/m |
| Pinocembrin (HPLC) : | ≥ 1,5 % m/m | 2,78 % m/m |
| Galangin (HPLC) : | ≥ 1,5 % m/m | 2,24 % m/m |
| Apigenin (HPLC): | 0,15 % m/m | 0,31 % m/m |
| Phenolcarbonsäuren | | |
| Kaffeesäure (HPLC) : | ≥ 0,2 % m/m | 0,47 % m/m |
| p-Cumarsäure (HPLC) : | ≥ 1,5 % m/m | 1,73 % m/m |
| Benzoesäure (HPLC) : | ≥ 0,8 % m/m | 1,34 % m/m |
| Zimtsäure (HPLC) : | ≥ 0,2 % m/m | 0,36 % m/m |
| | ≥ 1,3 % m/m | 1,43 % m/m |
| Schwermetalle | Arzneimittel-Kontaminanten-Empfehlung vom 17.10.1991 | entspricht |
| Pestizid-Rückstände | Ph. Eur. 2.8.13 | entspricht |
| Amitraz | Bestimmungsgrenze: 0,05 mg/kg | ≤ 0,05 mg/ko |
| Anmerkung: | Ph. Eur. in der jeweils aktuellen Version | |

Der so hergestellte Extrakt wurde nach herkömmlichen Methoden in vitro gegen Herpes-simplex-Viren vom Typ 1 und 2 (HSV-1 und HSV-2) untersucht und seine antivirale Wirkung bestätigt.

In vitro wurde ebenfalls die antivirale Wirkung des Extraktes gegen Varizellen-/Zoster-Viren (VZV) bestätigt.

Dermatologischen Untersuchungen bei an Herpes labiales erkrankten Personen verwendeten Lippenbalsame mit einem Gehalt von 0,1; 0,5; 1,0 bzw. 2,0 Gew.-%. Alle Wirkstoffkonzentrationen erzielten hochsignifikante therapeutische Ergebnisse und bestätigten die Wirkungsstärke des erfindungsgemäßen Propolis-Extraktes.

## Patentansprüche

1. Verfahren zur Herstellung eines Extraktes aus einem Propolis-Rohstoff, umfassend die folgenden Schritte:
a) Extraktion des Propolis-Rohstoffes mit einem organischen Lösungsmittel enthaltenden Extraktionsmittel bei einer gegenüber der Raumtemperatur erhöhten Temperatur **oberhalb von 25 °C,**
b) Absenkung der Temperatur der in Schritt a) erhaltenen Lösung auf Raumtemperatur im Bereich von 15 °C bis 25 °C,
c) Abtrennung der in Schritt b) gebildeten unlöslichen Stoffe bei Raumtemperatur,
d) Absenkung der Temperatur der in Schritt c) erhaltenen Lösung auf eine Temperatur **unterhalb von 0 °C,**
e) Abtrennung der in Schritt d) gebildeten unlöslichen Stoffe, und
f) teilweise oder vollständige Entfernung des Extraktionsmittels.

2. Verfahren nach Anspruch 1, **dadurch gekennzeichnet, dass** man als Extraktionsmittel ein organisches Lösungsmittel mit einem verzweigten oder unverzweigten Alkohol einer Kettenlänge von 1 bis 5 C-Atomen einsetzt.

3. Verfahren nach Anspruch 2, **dadurch gekennzeichnet, dass** man die Extraktion mit einem Ethanol/Wasser-Gemisch durchführt, welches insbesondere einen Ethanol-Gehalt in einem Bereich von 10 bis 90 Vol.% aufweist.

4. **Verfahren nach Anspruch 3, dadurch gekennzeichnet, dass man die Extraktion bei einer Temperatur von 30 bis 50 °C durchführt.**

5. Verfahren nach einem der Ansprüche 1 bis 3, **dadurch gekennzeichnet, dass** man die Temperatur in Schritt d) in einem Bereich von -5 bis -30 °C einstellt.

6. Verfahren nach einem der Ansprüche 1 bis 4, **dadurch gekennzeichnet, dass** man in Schritt d) die Absenkung der Temperatur über ein durchgehendes oder unterbrochenes Zeitintervall von 1 bis 48 h aufrechterhält.

7. Verfahren nach einem der Ansprüche 1 bis 5, **dadurch gekennzeichnet, dass** man die in den Schritten b) und d) gebildeten unlöslichen Stoffe abfiltriert.

8. Verfahren nach einem der Ansprüche 1 bis 6, **dadurch gekennzeichnet, dass** man die Entfernung des Extraktionsmittels bei einem Druck unterhalb 500 mbar, insbesondere unterhalb 150 mbar durchführt.

9. Propolis-Extrakt erhältlich nach dem Verfahren nach einem der Ansprüche 1 bis 7 zur Behandlung viraler Erkrankungen und/oder Entzündungen, insbesondere Herpes-Erkrankungen.

10. Pharmazeutische Zusammensetzungen enthaltend einen Propolis-Extrakt nach Anspruch 8.

11. Zusammensetzungen nach Anspruch 10, **dadurch gekennzeichnet, dass** es sich um ein Gel, eine Lotion, eine Emulsion, einen Lippenbalsam oder eine Crème handelt.

12. Zusammensetzungen nach einem der Ansprüche 10 oder 11, **dadurch gekennzeichnet, dass** sie den Extrakt in einer Menge von 0,01 bis 3,0 Gew.-%, insbesondere von 0,5 Gew.-% enthalten.

## Claims

1. A process for preparing an extract from a propolis raw material, comprising the following steps:
a) extracting the propolis raw material with an extractant containing an organic solvent at a temperature that is elevated with respect to room temperature and above 25°C;
b) lowering the temperature of the solution obtained in step a) to room temperature within a range of from 15 °C to 25 °C;
c) separating the insoluble materials formed in step b) at room temperature;
d) lowering the temperature of the solution obtained in step c) to a temperature below 0 °C;
e) separating the insoluble materials formed in step d); and
f) partially or completely removing the extractant.

2. The process according to claim 1, **characterized in that** an organic solvent comprising a branched or unbranched alcohol with a chain length of 1 to 5 carbon atoms is employed as the extractant.

3. The process according to claim 2, **characterized in that** said extraction is performed with an ethanol/water mixture, especially one having an ethanol content within a range of from 10 to 90% by volume.

4. The process according to claim 3, **characterized in that** said extraction is performed at a temperature from 30 to 50 °C.

5. The process according to any of claims 1 to 3, **characterized in that** the temperature in step d) is set within a range of from -5 to -30 °C.

6. The process according to any of claims 1 to 4, **characterized in that** the lowered temperature in step d) is maintained over a continuous or discontinuous period of time of from 1 to 48 hours.

7. The process according to any of claims 1 to 5, **characterized in that** the insoluble materials formed in steps b) and d) are filtered off.

8. The process according to any of claims 1 to 6, **characterized in that** said removing of the extractant is performed under a pressure of below 500 mbar, especially below 150 mbar.

9. A propolis extract obtainable by the process according to any of claims 1 to 7 for the treatment of viral diseases and/or inflammations, especially herpes diseases.

10. Pharmaceutical compositions containing a propolis extract according to claim 8.

11. The compositions according to claim 10, **characterized by** being a gel, a lotion, an emulsion, a lip balm, or a cream.

12. The compositions according to either of claims 10 or 11, **characterized by** containing said extract in an amount of from 0.01 to 3.0% by weight, especially 0.5% by weight.

## Revendications

1. Procédé pour la production d'un extrait d'une matière première de propolis, comprenant les étapes consistant à :
a) extraire la matière première de propolis avec un agent d'extraction contenant un solvant organique à une température supérieure à la température ambiante et supérieure à 25 °C,
b) abaisser la température de la solution obtenue à l'étape a) à la température ambiante comprise entre 15 °C et 25 °C,
c) séparer à la température ambiante les substances insolubles formées à l'étape b),
d) abaisser la température de la solution obtenue à l'étape c) à une température inférieure à 0 °C,
e) séparer les substances insolubles formées à l'étape d), et
f) éliminer l'agent d'extraction partiellement ou complètement.

2. Procédé selon la revendication 1, **caractérisé en ce qu'**un solvant organique comprenant un alcool ramifié ou non ramifié avec une longueur de chaîne de 1 à 5 atomes de carbone est utilisé comme agent d'extraction.

3. Procédé selon la revendication 2, **caractérisé en ce que** ladite extraction est effectuée avec un mélange éthanol/eau, notamment ayant une teneur en éthanol comprise entre 10 et 90% en volume.

4. Procédé selon la revendication 3, **caractérisé en ce que** ladite extraction est effectuée à une température de 30 à 50 °C.

5. Procédé selon l'une quelconque des revendications 1 à 3, **caractérisé en ce que** la température à l'étape d) est réglée dans une plage de -5 à -30 °C.

6. Procédé selon l'une quelconque des revendications 1 à 4, **caractérisé en ce que** ledit abaissement de la température à l'étape d) est maintenu pendant une période continue ou discontinue de 1 à 48 heures.

7. Procédé selon l'une quelconque des revendications 1 à 5, **caractérisé en ce que** les substances insolubles formées aux étapes b) et d) sont éliminées par filtration.

8. Procédé selon l'une quelconque des revendications 1 à 6, **caractérisé en ce que** ladite élimination de l'agent d'extraction est effectuée sous une pression inférieure à 500 mbar, notamment inférieure à 150 mbar.

9. Extrait de propolis pouvant être obtenu par le procédé selon l'une quelconque des revendications 1 à 7, destiné au traitement des maladies virales et/ou des inflammations, notamment des maladies à herpèsvirus.

10. Compositions pharmaceutiques contenant un extrait de propolis selon la revendication 8.

11. Compositions selon la revendication 10, **caractérisées par** le fait d'être un gel, une lotion, une émulsion, un baume aux lèvres ou une crème.

12. Compositions selon l'une quelconque des revendications 10 ou 11, **caractérisées par** le fait de contenir ledit extrait en une quantité de 0,01 à 3,0% en poids, notamment 0,5% en poids.
